# EUROPEAN PATENT APPLICATION

(11) **EP 2 042 870 A1**
(43) Date of publication of application: **01.04.2009**
(21) Application number: 08016700.0
(22) Date of filing: 23.09.2008
(51) Int. Cl.: G01N 33/543

(54) **Method of high sensitive immunoassay**

(30) Priority: 28.09.2007 JP 2007254179
(71) Applicant: Fujifilm Corporation, Tokyo 106-0031 (JP)
(72) Inventor: Muraya, Koji, Saitama 351-8585 (JP); Saito, Yoshio, Saitama 351-8585 (JP); Ohara, Tomoya, Kanagawa 258-8577 (JP)
(74) Representative: Beckmann, Claus

(57) **Abstract**

An object of the present invention is to provide a method of high sensitive immunoassay using immunoagglutination reaction by antigen-antibody reaction for quantification of a biological substance. The present invention provides a method for assaying an antigen comprising assaying agglutination which is generated by contacting an antigen with a carrier on which antibodies are supported, wherein the carrier on which antibodies are supported comprises a plurality of types of monoclonal antibodies that recognize different epitopes of an antigen supported thereon via simultaneous sensitization to the carrier.

## Description

### Technical Field

The present invention relates to a method of immunoassay comprising performing an agglutination reaction using an immunological material, such as an antigen or antibody, and a carrier, such as latex, to thereby assay an antigen. More particularly, the present invention relates to a method of high sensitive immunoassay using an antibody-supported carrier that is obtained by simultaneously sensitizing a plurality of types of monoclonal antibodies reacting with different epitopes to the carrier.

### Background Art

In the past, a disease has been identified through clinical diagnosis by sampling a body fluid component, such as blood (e.g., whole blood, blood plasma, or blood serum) or urine, using such body fluid component as an analyte, quantifying a concentration of a given component in the analyte, and employing the concentration of such given component as an indicator. When an assay target component in the analyte is an antigen or antibody in such clinical test settings, an immunodiagnostic reagent has been used as a means for quantifying the component with the utilization of properties of an antibody to strictly distinguish and bind to an antigen.

When using the antigen-antibody reaction-based immunodiagnostic reagent, methods that do not involve the use of a labeling reaction, i.e., non-labeling methods, such as the precipitation reaction-based immunodiffusion, immunoturbidimetry, and immunonephelometry, the agglutination reaction-based hemagglutination or latex method and the like may be employed. To date, enzyme immunoassay (EIA), radioimmunoassay (RIA), fluorescence immunoassay (FIA), chemiluminescence immunoassay (CLIA), bioluminescence immunoassay (BLIA), and other techniques have been employed as methods that involve the use of a labeling reaction, in accordance with types or properties of materials to be labeled.

In the EIA method, for example, an enzyme reaction is allowed to proceed for a long period of time to accumulate (i.e., amplify) the reaction product, and a signal emitted from enzyme is then detected. Thus, a very sensitive immune-reactive reagent can be prepared. This applies to the other labeling techniques. Such technique, however, requires labeling of an antibody or antigen bound to a target antigen or antibody as an assay target component with an enzyme (e.g., peroxidase, β-galactosidase, or alkaline phosphatase). This complicates preparation of a reagent. Labeling techniques, including the EIA method, are generally classified into homogeneous techniques that do not involve B/F separation and heterogeneous techniques that involve B/F separation. The heterogeneous technique is particularly excellent in terms of sensitivity and safety because of B/F separation; however, the heterogeneous technique is inferior to the homogeneous technique in terms of promptness and convenience.

Further, labeling techniques, such as the EIA or RIA method, necessitate complicated assay operations and use of a special assay apparatus, equipment, facilities, and the like. Manufacturers of labeling-based immunodiagnostic reagents have overcome such drawbacks by supplying relatively large automated analyzers, which are developed exclusively for their own reagents. This consequently makes extensive-spreading of immunodiagnostic reagents and systems difficult in the POCT field.

Thus, labeling techniques are advantageous in terms of sensitivity and safety; however, such techniques are disadvantageous in terms of promptness and convenience. Further, labeling techniques necessitate complicated assay operations and use of a special assay apparatus, equipment, facilities, and the like. Accordingly, labeling techniques can be relatively easily employed at moderate- to large-scale hospitals equipped with a central laboratory where clinical technologists are always present. At small-scale medical facilities, such as small-scale hospitals, practitioners, clinics, or veterinary hospitals, or in the POCT field, however, employment of such techniques still remains difficult.

In the case of non-labeling techniques typified by the latex method, a process of labeling is unnecessary. Also, photometry at a given wavelength in the ultraviolet, visible, and near-infrared regions enables quantification of assay target components. Thus, provision of general and small spectroscopic apparatus is sufficient. Accordingly, it is easily deduced that employment of non-labeling techniques in the small-scale medical facilities or in the POCT field is easy.

While non-labeling techniques are advantageous in terms of promptness and convenience, the aforementioned amplification effects of the labeling techniques cannot be attained in principle. Thus, non-labeling techniques are disadvantageous in terms of sensitivity and safety. From a different point of view, this indicates that improvement in sensitivity of a non-labeling technique, such as the latex method, enables easy introduction of immunodiagnostic reagents and systems into small-scale medical facilities or the POCT field.

In particular, an agglutination reaction via the latex method is excellent in terms of promptness, convenience, and general versatility in the following respects. That is, a method for preparing a reagent is relatively easy. A user is only required to add an analyte to the latex reagent to initiate an assay, assay may be carried out at a given wavelength in the ultraviolet, visible, and near-infrared regions for a given period of time, and assay is generally completed within several minutes to several tens of minutes.

As a technique of supersensitization in conventional latex agglutination techniques, addition of a given sensitizer (agglutination promoter), such as polyvinyl pyrrolidone, polyethylene glycol, dextran, or dextran sulfate, disclosed in, for example, JP Patent Publication (kokai) No. H05-80051 A (1993), H05-297002 A (1993), or H06-58935 A (1994) is available. Although such sensitizer is effective for increasing assay sensitivity and a signal intensity, such sensitizer would also increase non-specific reactions disadvantageously. A method of defining diameters of latex particles to be used in a given range, a method of allowing the presence of latex particles having different diameters, and a method of adding a surfactant disclosed in JP Patent Publication (kokai) Nos. S60-53846 A (1985), H01-118770 A (1989), and H02-257063 A (1990) are disadvantageous in the following respect. As a latex particle diameter becomes larger, sensitivity is increased, but the range of quantification becomes narrower. In the presence of latex particles having different diameters, the effects of an individual particle diameter are limited. When a surfactant is to be added, the effects of sensitivity improvement are insufficient.

In the method of treating an antibody with a reducing agent to fragmentize the antibody and allowing the fragmented antibody to be supported on a carrier such as latex disclosed in JP Patent Publication (kokai) No. 2000-206115 A, pretreatment of an antibody is required. This sacrifices convenience of preparation of a reagent, which is an advantage of the latex agglutination method.

JP Patent Publication (kokai) No. H10-90268 A (1998) discloses an immunological particle agglutination reaction using both a polyclonal antibody and a monoclonal antibody as antibodies reacting with an assay target component. However, this technique is not intended for supersensitization, i.e., improvement in a response of an assay target component at a low concentration region. This technique is intended to inhibit the prozone phenomenon at a high concentration region and to enable assay in an extensive dynamic range even with the use of an apparatus without a mechanism of diluting an analyte. In this method, a polyclonal antibody may be used in combination with a monoclonal antibody. Also, such antibodies may be immobilized independently on an insoluble carrier particle, and then the resultants may be mixed. Alternatively, a mixture of antibodies may be immobilized on an insoluble carrier particle.

### Disclosure of the Invention

An object of the present invention is to provide a method for specifically and selectively enhancing the agglutination reaction of analyte components without deterioration of the advantage of a latex agglutination method (i.e., convenience of preparation of a reagent), which could not be achieved by a conventional method for supersensitizing an immunodiagnostic reagent.

The present inventors have conducted concentrated studies in order to attain the above object. As a result, they succeeded in specifically and selectively enhancing the agglutination reaction of target analyte components while maintaining convenience of preparation of a reagent by preparing an antibody solution comprising the previously mixed plurality of types of antibodies and using the resulting antibody solution to allow the antibody to be supported on latex, instead of individual supporting of antibodies separately on latex and mixing them at the time of the agglutination reaction, when preparing a latex reagent using a plurality of types of monoclonal antibodies that recognize different epitopes of antigens. This has led to the completion of the present invention.

Thus, the present invention provides a method for assaying an antigen comprising assaying agglutination which is generated by contacting an antigen with a carrier on which antibodies are supported, wherein the carrier on which antibodies are supported comprises a plurality of types of monoclonal antibodies that recognize different epitopes of an antigen supported thereon via simultaneous sensitization to the carrier.

Preferably, the carrier on which antibodies are supported comprises a mixture of a plurality of monoclonal antibodies that recognize different epitopes of an antigen at an arbitrary mixing ratio via sensitization to the carrier.

Preferably, the antigen is CRP (C-reactive protein) or TSH (thyroid stimulating hormone).

Another aspect of the present invention provides a reagent for immunoassay used for the method of the present invention, which comprises a carrier which is prepared via simultaneous sensitization and supporting of a plurality of monoclonal antibodies that recognize different epitopes of an antigen.

The present invention provides a method for assaying an antigen that specifically and selectively enhances the agglutination reaction of the target analyte components while maintaining convenience of preparation of a reagent. The present invention also provides a reagent for immunoassay used for the above method.

### Brief Description of the Drawing

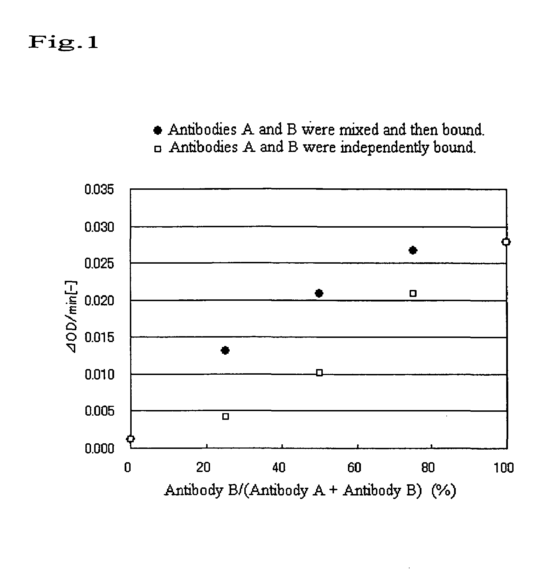

Fig. 1 shows a response of a latex reagent to which two types of antibodies had been bound and a standard CRP solution. For comparison, a response of a latex reagent to which a polyclonal antibody had been bound and a standard CRP solution is shown by broken line.

### Best Modes for Carrying out the Invention

Hereafter, the present invention is described in detail.

The method for assaying an antigen of the present invention comprises assaying agglutination which is generated by contacting an antigen with a carrier on which antibodies are supported, wherein the carrier on which antibodies are supported comprises a plurality of types of monoclonal antibodies that recognize different epitopes of an antigen supported thereon via simultaneous sensitization to the carrier. The term "assay of an antigen" used herein should be understood in the broadest possible way, including detection of the presence or absence of an antigen and assay of the antigen amount (i.e., quantification). Also, the term "assay of agglutination" used herein is not limited to assay of the degree of agglutination. It should be understood in a broad way, including, for example, observation of agglutination.

An antibody that can be used in the present invention may be any monoclonal antibody without particular limitation, provided that a plurality of types of antibodies that recognize different epitopes of the target analyte components are included.

Also, animal species of the antibody are not particularly limited. For example, a monoclonal antibody derived from a mouse, rat, rabbit, goat, sheep, or human can be used. Further, a modified antibody such as a chimeric antibody, a commercialized antibody, or an antibody prepared from the animal blood serum or culture supernatant in accordance with a conventional technique can be used.

In the present invention, preferably, a mixture comprising a plurality of types of monoclonal antibodies that recognize different epitopes of the antigen at any mixing ratio may be sensitized to a carrier, so that the antibody can be supported on the carrier. When mixing a plurality of types of antibodies, such plurality of types of antibodies can be mixed at any mixing ratio without particular limitation. In such a case, agglutination of target analyte components can be selectively and specifically enhanced by allowing a solution of previously mixed antibodies to be supported on latex, instead of individual supporting of antibodies separately on latex and mixing them at the time of the agglutination reaction.

In the present invention, known carriers can be used without particular limitation, provided that such carriers can support an antibody thereon, and the antibody-supporting carrier can cause the agglutination reaction resulting from an antigen-antibody reaction in the presence of an antigen. The amounts of antibodies and carriers used for preparing carriers on which antibodies are supported are not particularly limited, and any amounts can be employed.

Conventional polystyrene particles can be used as carrier. The material of such particle is not particularly limited, provided that such material is a known material for a carrier used in the field of immunoagglutination reaction. Examples of such materials include synthetic polymer particles, such as polystyrene, a styrene-methacrylic acid copolymer, a styrene-glycidyl (meth)acrylate copolymer, a styrene-styrene sulfonate copolymer, a methacrylic acid polymer, an acrylic acid polymer, an acrylonitrile-butadiene-styrene copolymer, a vinyl chloride-acrylic acid ester copolymer, and a polyvinyl acetate-acrylate copolymer. A latex suspension comprising such particles homogeneously suspended therein is preferable. Further examples include other organic polymer powders, inorganic powders, microorganisms, blood cells or pieces of cell membrane, and a plastic microtiter plate. Examples of inorganic powders include metal pieces of gold, titanium, and nickel, silica, and alumina.

The diameters of such carrier particles vary depending on the material of a carrier, the concentration range at which an assay target component is quantified, the assay equipment, the method, or the like. In general, a diameter is 0.01 to 1.0 µm, and more preferably 0.05 to 0.7 µm.

When preparing a reagent by allowing a carrier to support an antibody thereon, any known method for preparing a reagent for immunoagglutination as disclosed in, for example, JP Patent Publication (kokai) No. 2000-206115 A can be used. An antibody can be supported on a carrier via physical adsorption or chemical binding via covalent binding. After an antibody is supported on a carrier, a surface of a carrier that is not covered by an antibody can be coated with a known blocking agent, such as bovine serum albumin (BSA), Block Ace, skimmed milk, or casein. Such blocking agent may be subjected to pretreatment such as partial denaturation with heat, acid, alkali, or the like, according to need.

An example of a specific method for preparing a reagent by allowing a carrier to support an antibody thereon is described below. To a solution comprising latex particles dispersed at a solid concentration of 0.1% to 10% therein, an antibody solution adjusted at a concentration of 0.01 to 20 mg/ml is added, and the resultant is then mixed. The mixture is continuously agitated at 4°C to 50°C for 10 minutes to 48 hours. Thereafter, a solution is separated from carriers via centrifugation or other means to thoroughly remove antibodies that did not bind to carriers from the solution. Thereafter, carriers are repeatedly washed with buffer 0 to 10 times. Carriers are mixed with antibodies to bind the antibodies to the carriers, and part of the carrier surface to which no antibodies have been bound is then preferably protected with a component that is not associated with an antigen-antibody reaction, preferably a protein, and more preferably a blocking agent, such as bovine serum albumin (BSA), Block Ace, skimmed milk, or casein, for the purpose of preventing a non-specific reaction at the time of assay.

Target analytes may be any fluid containing a biogenic component. Examples thereof that can be used include blood (whole blood, blood plasma, or blood serum), pleural fluid, ascites, lymph fluid, urine, feces, sweat, and spinal fluid. A biogenic component (antigen) as an analyte target may be any substance, provided that an antigen-antibody reaction can be carried out. Examples thereof include immunoglobulin, another protein or peptide, a polysaccharide, a saccharide, lipid, hormone, hormone-like substance, and drug.

According to the assay method of the present invention, a dispersion of an antibody-supporting carrier is brought into contact with an analyte containing an assay target component to initiate the reaction, and generation of aggregates resulting from the antigen-antibody reaction is quantified by assay at a given wavelength in the ultraviolet, visible, or near-infrared region for a given period of time. In general, assay is completed within several minutes to several hours. The reaction may be initiated with the use of two solutions, i.e., a carrier dispersion and an analyte solution. Further, an additional solution containing a stabilizer or agglutination promoter may be prepared, and the reaction may proceed with the use of three solutions. Such stabilizer or agglutination promoter may be contained in a carrier dispersion or analyte solution, and such substance may be added according to need.

A stabilizer is not particularly limited, provided that it would stabilize the reagents and components used for the reaction or the progress of reaction. Examples thereof include a buffer, such as Good's buffer, a synthetic or natural polymer, such as polyethylene glycol or a polysaccharide, and a surfactant. This applies to a case in which such substance functions as a promoter of an agglutination reaction, as well as a stabilizer. The reagents and components used for the reaction refer to, for example, an antibody that has been bound to a carrier or an assay target component. As described in JP Patent Publication (kokai) No. H08-187095 A (1996), for example, addition of a saccharide for the purpose of stabilizing a protein such as an antibody or enzyme is known. Accordingly, copresence of a stabilizer, such as a saccharide or amino acid, for the purpose of stabilizing an antibody in the reagent, is easy to deduce. When an assay target component is an unstable substance such as a protein, a stabilizer may be allowed to be present for the purpose of maintaining the assay target stably during the assay.

Examples of an agglutination promoter include a synthetic or natural polymer, such as polyethylene glycol, polyglycosyl methacrylate, polyvinyl pyrrolidone, carboxy methyl cellulose, dextran, and pullulan. An example of a surfactant having similar effects is a polyethylene glycol fatty acid monoester derivative disclosed in JP Patent Publication (kokai) No. H05-297002 A (1993). A fatty acid constituting a hydrophobic portion may be saturated or unsaturated, and specific examples thereof include lauric acid, myristic acid, palmitic acid, stearic acid, arachic acid, behenic acid, oleic acid, linoleic acid, and linolenic acid.

A buffer that can be used may have a buffering capacity at a pH level at which a general antigen-antibody reaction is carried out. Such pH level is 5 to 11, and more preferably 6 to 10. Any buffer that can be used in general biochemical experiment, such as buffer capable of buffering at the required pH levels, can be used. Examples thereof include phosphate buffer, Tris buffer, MES buffer, HEPES buffer, glycine buffer, citrate buffer, and Good's buffer. Also, fluid associated with the agglutination reaction, such as a dispersion of antibody-supporting carriers or an analyte comprising an assay target component, can comprise a surfactant, a synthetic or natural polymer, an organic or inorganic reagent, or the like, according to need. For example, any known surfactant for an immunoagglutination reaction, such as a nonionic, anionic, or cationic surfactant, can be used. The same applies to other reagents.

When an agglutination reaction resulting from an antigen-antibody reaction is to be performed, the degree of agglutination can be assayed visually, via filming with the use of a video or other means, or spectroscopically. Spectroscopic assay may be carried out in accordance with a known technique with the use of a solution comprising a dispersion of antibody-supporting carriers in contact with an analyte containing an assay target component. For example, diameters of carrier particles, concentration, or changes (increase or decrease) in the intensity of scattered light, absorbance, or transmitted beam with the elapse of a reaction time may be assayed.

Hereafter, an embodiment of a reagent for latex agglutination is described as an example of the present invention, although the technical scope of the present invention is not limited thereto.

### Examples

### <Example 1> Detection of CRP(C-reactive protein)

### (1) Preparation of antibody-bound latex

Two types of antibodies; i.e., mouse-derived anti-CRP monoclonal antibody A and mouse-derived anti-CRP monoclonal antibody B, reacting with different epitopes, were prepared in accordance with a conventional technique. A method for preparing monoclonal antibodies is described in, for example, Handbook of Experimental Immunology, (NAKASHIMA, Izumi (ed.), the University of Nagoya Press). Specifically, CRP was used as an antigen to immunize a mouse, the spleen cells were removed therefrom several weeks later, and the spleen cells were fused with myeloma cells using polyethylene glycol. After culturing the fused cells in HAT selection medium for approximately two weeks, monoclonal antibody-producing clones were cloned and selected from anti-CRP antibody-producing hybridoma clones. The anti-CRP antibodies A and B that recognize different epitopes were purified therefrom and then used. The fact that anti-CRP antibodies A and B recognize different epitopes of CRP was verified via ELISA or other means. Polystyrene latex particles (particle diameter: 0.5 µm, JSR) were diluted with glycine buffer (pH 9.6) to bring a solid concentration to 3%. In a similar manner, the anti-CRP antibodies A and the anti-CRP antibodies B were adjusted at concentrations of approximately 2 mg/ml each. The equivalent amounts of anti-CRP antibodies A and anti-CRP antibodies B thus prepared were added to the latex dispersion, the resulting mixture was agitated, and the reaction was allowed to proceed at room temperature for 1 hour. Centrifugation was carried out at 11,000 rpm for 15 minutes, the supernatant was removed, and 0.2% BSA in glycine buffer (pH 9.6, containing 0.001% of Triton X-100) was added to the precipitate as a blocking agent to bring the solid latex concentration to 1.5%. After the resultant was agitated at room temperature for 1 hour, centrifugation was carried out at 11,000 rpm for 15 minutes, and the supernatant was removed. Glycine buffer (pH 9.6) was added to the precipitate for redispersion, centrifugation was carried out again at 11,000 rpm for 15 minutes, and the supernatant was removed. Glycine buffer (pH 9.6) was added to the precipitate for redispersion to a final solid latex concentration of 3.0% therein, and the resultant was designated as an anti-CRP antibody-bound latex reagent. In this case, two types of antibodies (anti-CRP monoclonal antibody A and anti-CRP monoclonal antibody B) were bound to latex individually, and the latexes were mixed at given ratios (100:0, 75:25, 50:50, 25:75, 0:100) as shown in Table 1. Alternatively, two types of antibodies were mixed in advance at given ratios (75:25, 50:50, 25:75), and the resulting mixtures were allowed to bind to latex, as shown in Table 1.

**Table 1 : Mixing ratio of antibody A to antibody B (A:B)**

| Method for preparing reagent | (1) | (2) | (3) | (4) | (5) |
|---|---|---|---|---|---|
| Antibody A and antibody B are independently bound | 100:0 | 75:25 | 50:50 | 25:75 | 0:100 |
| Antibody A is mixed with antibody B and the mixture was bound | - | 75:25 | 50:50 | 25:75 | - |

### (2) Detection of CRP

A standard CRP solution was diluted with buffer to bring the CRP concentration to 0 to 24 µg/dl, and the resultant was designated as the CRP solution (analyte). The anti-CRP antibody-bound latex shown in Table 1 was allowed to react with an analyte at 37°C, and changes in absorbance at given time points were assayed using a spectrophotometer (Hitachi, Ltd.). At the outset, 400 µl of the CRP solution diluted to a given concentration was added to the reaction cell, followed by preheating at 37°C for 5 minutes. Thereafter, 600 µl of the anti-CRP antibody-bound latex reagent, which had been previously diluted to a final solid latex content of 0.015% in the reaction solution, was added to initiate the reaction, and the reaction was terminated 40 minutes after the initiation of the reaction. During the reaction, the absorbance at 800 nm was assayed every given period of time to determine the response to CRP at a given concentration (2.4 µg/dl) based on changes in absorbance. The results of assay are shown in Fig. 1. The horizontal axis indicates the ratios of antibodies A to antibodies B, and the vertical axis indicates changes in OD values at 0.25 minutes and 1.93 minutes after the initiation of the reaction in terms of the changes per minute, Δ OD/min. A higher Δ OD/min. value indicates a stronger agglutination reaction. Regardless of the mixing ratio of antibody A to antibody B, an agglutination reaction was stronger when antibodies were mixed in advance and then bound to latex, as compared with when antibodies were independently bound to latex and the latexes were mixed at the time of reaction. This demonstrates that a higher sensitivity can be realized when antibodies are mixed in advance. Further, using an anti-CRP polyclonal antibody-bound latex reagent which was prepared for the purpose of comparison, the same procedure was performed. As a result, weaker agglutination reaction was observed as compared with the case of the latex reagent which was prepared by mixing monoclonal antibodies at a given ratio. Therefore, it was shown that a mixture of several types of monoclonal antibodies which recognize each specific epitome is more superior than a polyclonal antibody which is also a mixture.

### <Example 2> Detection of thyroid stimulating hormone (TSH)

### (1) Preparation of antibody-bound latex

Four types of the commercially available anti-TSH monoclonal antibody were purchased. The purchased anti-TSH monoclonal antibodies are anti-TSH monoclonal antibodies which are manufactured by Medix (Finland), and four types of Clone Numbers 5401, 5405, 5407 and 5409 were used. Among these four types anti-TSH antibodies of different clone numbers, the clone numbers 5405 and 5407 recognize the same epitope of TSH, and 5401, 5405/5407 and 5409 recognize the different epitope of TSH respectively. As to the combination A (5401, 5405 and 5409) and the combination B (5401, 5407 and 5409), anti-TSH antibodies were bound to a latex particle using the same method as that of Example 1. Namely, polystyrene latex particles (particle diameter: 0.5 µm, JSR) were diluted with glycine buffer (pH 9.6) to bring a solid concentration to 3%. In a similar manner, the anti-TSH antibodies 5401, 5405, 5407 and 5409 were adjusted at a concentration of approximately 2 mg/ml. The equivalent amounts of the mixture were added to the latex dispersion, the resulting mixture was agitated, and the reaction was allowed to proceed at room temperature for 1 hour. Centrifugation was carried out at 11,000 rpm for 15 minutes, the supernatant was removed, and 0.2% BSA in glycine buffer (pH 9.6, containing 0.001% of Triton X-100) was added to the precipitate as a blocking agent to bring the solid latex concentration to 1.5%. After the resultant was agitated at room temperature for 1 hour, centrifugation was carried out at 11,000 rpm for 15 minutes, and the supernatant was removed. Glycine buffer (pH 9.6) was added to the precipitate for redispersion, centrifugation was carried out again at 11,000 rpm for 15 minutes, and the supernatant was removed. Glycine buffer (pH 9.6) was added to the precipitate for redispersion to a final solid latex concentration of 3.0% therein, and the resultant was designated as an anti-TSH antibody-bound latex reagent. In this case, three types of anti-TSH monoclonal antibodies were bound to latex individually, and the latexes were mixed at an equal ratio (1:1:1), and separately three types of anti-TSH monoclonal antibodies were mixed in advance at an equal ratio (1:1:1) and the resulting mixture was allowed to bind to latex.

### (2) Detection of TSH

A standard TSH solution was diluted with buffer to bring the TSH concentration to 5 µg/dl, and the resultant was designated as a TSH solution (analyte). The anti-TSH antibody-bound latex was allowed to react with an analyte at 37°C, and changes in absorbance at given time points were assayed using a spectrophotometer (Hitachi, Ltd.). At the outset, 400 µl of the TSH solution diluted to a given concentration was added to the reaction cell, followed by preheating at 37°C for 5 minutes. Thereafter, 600 µl of the anti-TSH antibody-bound latex reagent, which had been previously diluted to a final solid latex content of 0.015% in the reaction solution, was added to initiate the reaction, and the reaction was terminated 40 minutes after the initiation of the reaction. The absorbance of the reaction cell at 800 nm after 40 minutes was assayed to determine the response to TSH at a given concentration (5 µg/dl). As the blank, the absorbance after 40 minutes in the case of TSH concentration of 0 µg/dl was assayed, and the difference between the absorbance of 5 µg/dl and the absorbance of 0 µg/dl was calculated. The results of assay is shown in Table 2. From this result, it was shown that it is more advantages that several antibodies each of which recognizes different epitope are mixed in advance at the time of preparation of latex reagent and then the mixture is bound to a latex, than that such several antibodies are bound to latex dependently and then the latex is mixed at the time of measurement, even in the case of not only anti-CRP antibody but also anti-TSH antibody.

**Table 2: Response of a combination of anti-TSH antibody to TSH: Reaction time 40 min. Difference between the absorbance (TSH concentration : 5 µg/dl) and the absorbance (TSH concentration: 0 µg/dl)**

| Combination of Antibody | ①(5401, 5405, 5409) | ②(5401, 5407, 5409) |
|---|---|---|
| Three types of antibody are independently bound | 0.263 | 0.157 |
| A mixture of three types of antibody is bound | 0.304 | 0.289 |

## Claims

1. A method for assaying an antigen comprising assaying agglutination which is generated by contacting an antigen with a carrier on which antibodies are supported, wherein the carrier on which antibodies are supported comprises a plurality of types of monoclonal antibodies that recognize different epitopes of an antigen supported thereon via simultaneous sensitization to the carrier.

2. The method for assaying an antigen according to claim 1, wherein the carrier on which antibodies are supported comprises a mixture of a plurality of monoclonal antibodies that recognize different epitopes of an antigen at an arbitrary mixing ratio via sensitization to the carrier.

3. The method for assaying an antigen according to claim 1, wherein the antigen is CRP (C-reactive protein) or TSH (thyroid stimulating hormone).

4. A reagent for immunoassay used for the method according to claim 1, which comprises a carrier which is prepared via simultaneous sensitization and supporting of a plurality of monoclonal antibodies that recognize different epitopes of an antigen.
